# EUROPEAN PATENT SPECIFICATION

(11) **EP 3 975 850 B1**
(45) Date of publication and mention of the grant of the patent: **12.07.2023**
(21) Application number: 21723994.6
(22) Date of filing: 12.05.2021
(51) Int. Cl.: A61B 6/00, A61B 5/00, G06T 7/20

(54) **X-RAY IMAGING SYSTEM**
RÖNTGENBILDGEBUNGSSYSTEM
SYSTÈME D'IMAGERIE PAR RAYONS X

(30) Priority: 19.05.2020 EP 20175453
(43) Date of publication of application: 06.04.2022
(73) Proprietor: Koninklijke Philips N.V., 5656 AG Eindhoven (NL)
(72) Inventor: MENSER, Bernd, 5656 AE Eindhoven (NL); RIBBING, Carolina, 5656 AE Eindhoven (NL); STEADMAN BOOKER, Roger, 5656 AE Eindhoven (NL)
(74) Representative: Philips Intellectual Property & Standards
(86) International application number: PCT/EP2021/062564
(87) International publication number: WO 2021/233748

(56) References cited:
- WO-A1-2020/044345
- US-A1- 2014 064 450
- US-A1- 2017 196 528
- US-A1- 2018 350 081

## Description

### FIELD OF THE INVENTION

The present invention relates to an X-ray imaging system, an apparatus for post-processing a radiograph X-ray attenuation image, an X-ray imaging method, a method apparatus of post-processing a radiograph X-ray attenuation image, as well as to a computer program element and a computer readable medium.

### BACKGROUND OF THE INVENTION

In contrast to CT (volume image) or fluoroscopy (temporal sequence of images) a radiography consists of only a single x-ray projection image, which is in contrast to CT (where a volume image is acquired) or fluoroscopy (where a temporal sequence of images is acquired). However, patient motion during the image acquisition can cause motion blur, especially for examinations with long exposure times and where the patient moves rapidly.

Thus, large or fast patient motion during the X-ray integration window, also termed the acquisition time, in radiographic imaging leads to blurring of anatomical structures (motion blur). Unpredicted patient motion often occurs in imaging of non-cooperating patients, e.g. in paediatric radiography. Motion blur degrades the image quality and can impact the diagnostic value of an image. US 2014/064450 A1 discloses to calculate local motion vectors and a body motion index value in order to determine patient motion during the image acquisition. In addition, a body motion detection unit may be provided which obtains imaging information from the console, which controls the entire operation of the radiographic imaging apparatus. This imaging information may be information about the imaging conditions, e.g. imaging time, imaging interval, or whether or not the patient is secured during imaging, and also patient information.

Post-processing through de-blurring image processing has been an active research topic in the computer vision and image processing community, where the blurred X-ray image is interrogated in order to estimate a blurring kernel of a de-blurring algorithm.

However, there is a need to improve such post-processing to compensate for patient motion.

### SUMMARY OF THE INVENTION

It would be advantageous to improve the technology for compensating for patient movement in X-ray projection imaging.

The object of the present invention is solved with the subject matter of the independent claims, wherein further embodiments are incorporated in the dependent claims. It should be noted that the following described aspects and examples of the invention apply also to the X-ray imaging system, to the X-ray imaging method, as well as for the computer program element and computer readable medium.

According to a first aspect, there is provided an X-ray imaging system, comprising:
- a radiograph X-ray attenuation image acquisition unit;
- at least one sensor; and
- a processing unit.

The radiograph X-ray attenuation image acquisition unit is configured to acquire a radiograph image of a patient, wherein the radiograph image consists of only a single x-ray projection image. The radiograph X-ray attenuation image acquisition unit is configured to provide the radiograph image to the processing unit. The at least one sensor is configured to acquire sensor data of the patient. The at least one sensor is configured to provide the sensor data to the processing unit. The processing unit is configured to determine a magnitude and direction of movement of the patient during a time of acquisition of the radiograph image, the determination comprising utilization of the sensor data. The processing unit is configured to post-process the radiograph image comprising utilization of the determined magnitude and direction of movement of the patient during the time of acquisition of the radiograph image.

In this way, de-blurring and sharpening algorithms, for example, can utilize the patient movement data to improve the acquired radiograph and mitigate the effects of the patient having moved during the acquisition of the radiograph. It is to be noted that a radiograph can also be termed a radiogram.

Thus local motion information of the patient, as captured by an optical camera (or other sensor or sensors) can be used, for example, to guide the selection of sharpening operations in an image post-processing step in X-ray image acquisition, where temporal information from the camera (or other sensor or sensors) is used to quantify the amount of local motion in the scene at the time of the X-ray image acquisition.

In an example, the at least one sensor comprises one or more of: a 3D camera; a camera; a pair of stereo cameras; one or more ultrasound sensors; one or more radar sensors; one or more accelerometers attached to the patient.

In an example, the sensor data comprises sensor data of the patient during the time of acquisition of the radiograph image of the patient.

Thus, sensor data does not have to be acquired during the time of acquisition. For example, it could be acquired either side of the time of acquisition and by interpolation an indication of the sensor data that would have been acquired during the acquisition time determined. Or sensor data could be on one side of the acquisition time and extrapolated to the time of acquisition. Or indeed, sensor data acquired continuously on a periodic basis and a determination made as to the data that would have been acquired during the acquisition time through fitting a piecewise continuous model to the data. However, the sensor(s) can be triggered and/or data acquired from them during the actual acquisition time in order to provide real sensor data during the acquisition time. This data during the acquisition time can then be augmented by knowledge of the overall time sequence of sensor data in order to improve signal to noise of the sensor data.

In an example, the sensor data of the patient comprises a plurality of sensor data acquired at different time points. The processing unit is configured to determine the magnitude and direction of movement of the patient during the time of acquisition of the radiograph image. This can comprise interpolation and/or extrapolation of the plurality of sensor data.

Thus sensor data can be interpolated or extrapolated to determine the direction and magnitude of the patient's motion during the acquisition time and there need not be sensor data itself during that acquisition time, but such data during the acquisition time can be used in determining the direction and magnitude of the patient's movement.

In an example, post-processing of the radiograph image comprises a processing of the radiograph image by an image processing algorithm implemented by the processing unit. The processing unit is configured to modify at least one parameter of the image processing algorithm on the basis of the determined magnitude and direction of movement of the patient during the time of acquisition of the radiograph image.

In an example, post-processing of the radiograph image comprises a processing of the radiograph image by an image processing algorithm implemented by the processing unit. The processing unit is configured to select at least one parameter of the image processing algorithm on the basis of the determined magnitude and direction of movement of the patient during the time of acquisition of the radiograph image.

In an example, the image processing algorithm comprises a noise reduction algorithm, an image sharpening algorithm, a contrast enhancement algorithm, or a de-blurring algorithm.

In an example, the at least one parameter comprises a local strength and direction of at least one kernel of the image processing algorithm.

In an example, the processing unit is configured to estimate 2D optical flow or 3D optical flow in the image plane to determine the magnitude and direction of movement of the patient during the time of acquisition of the radiograph image.

In an example, post-processing of the radiograph image comprises a determination of a quantitative length value through the patient.

In an example, post-processing of the radiograph image comprises a determination of a quantitative volume value of the patient.

In an example, the system is configured to output information that the patient has moved during the time of acquisition of the radiograph image.

In an example, the system is configured to output the information that the patient has moved during the time of acquisition of the radiograph image when the determined magnitude of movement exceeds a threshold value.

According to a second aspect, there is provided an apparatus for post-processing a radiograph X-ray attenuation image, comprising:
- a processing unit.

The processing unit is configured to receive a radiograph image of a patient, wherein the radiograph image consists of only a single x-ray projection image, acquired by a radiograph X-ray attenuation image acquisition unit. The processing unit is configured to receive sensor data of the patient acquired by at least one sensor. The processing unit is configured to determine a magnitude and direction of movement of the patient during a time of acquisition of the radiograph image. The determination of the magnitude and direction of movement of the patient comprises utilization of the sensor data. The processing unit is configured to post-process the radiograph image comprising utilization of the determined magnitude and direction of movement of the patient during the time of acquisition of the radiograph image.

According to a third aspect, there is provided an X-ray imaging method, comprising:
a) acquiring a radiograph image of a patient, wherein the radiograph image consists of only a single x-ray projection image, with an radiograph X-ray attenuation image acquisition unit;
b) providing the radiograph image to a processing unit;
c) acquiring sensor data of the patient with at least one sensor;
d) providing the sensor data to the processing unit;
e) determining by the processing unit a magnitude and direction of movement of the patient during a time of acquisition of the radiograph image, the determining comprising utilizing the sensor data; and
f) post-processing by the processing unit the radiograph image comprising utilizing the determined magnitude and direction of movement of the patient during the time of acquisition of the radiograph image.

According to a fourth aspect, there is provided a method of post-processing a radiograph X-ray attenuation image, comprising:
b) providing a radiograph image of a patient, wherein the radiograph image consists of only a single x-ray projection image, to a processing unit, wherein the radiograph image of the patient was acquired by a radiograph X-ray attenuation image acquisition unit;
d) providing sensor data of the patient to the processing unit, wherein the sensor data was acquired by at least one sensor;
e) determining by the processing unit a magnitude and direction of movement of the patient during a time of acquisition of the radiograph image, the determining comprising utilizing the sensor data; and
f) post-processing by the processing unit the radiograph image comprising utilizing the determined magnitude and direction of movement of the patient during the time of acquisition of the radiograph image.

According to another aspect, there is provided a computer program element controlling a system as previously described which, if the computer program element is executed by a processing unit, is adapted to perform the method steps as previously described.

According to another aspect, there is provided a computer readable medium having stored computer element as previously described.

The computer program element, can for example be a software program but can also be a FPGA, a PLD or any other appropriate digital means.

Advantageously, the benefits provided by any of the above aspects equally apply to all of the other aspects and vice versa.

The above aspects and examples will become apparent from and be elucidated with reference to the embodiments described hereinafter.

### BRIEF DESCRIPTION OF THE DRAWINGS

Exemplary embodiments will be described in the following with reference to the following drawings:
Fig. 1 shows a schematic set up of an example of an X-ray imaging system;
Fig. 2 shows an X-ray imaging method; and
Fig. 3 shows an schematic representation of an image of a patient and regions of interest ROIs of the patient's back for which patient motion is determined from 3D camera images, and shows a plot of the determined movement value of the ROIs.

### DETAILED DESCRIPTION OF EMBODIMENTS

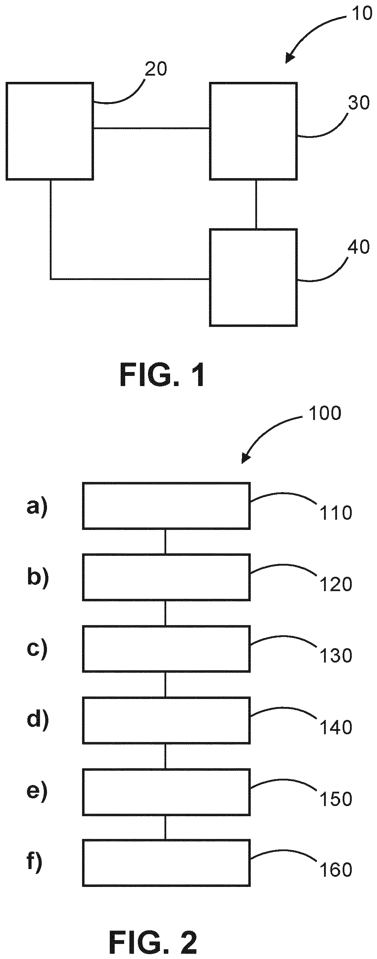

Fig. 1 shows schematically an example of an X-ray imaging system 10. The system 10 comprises a radiograph X-ray attenuation image acquisition unit 20, at least one sensor 30, and a processing unit 40. The radiograph X-ray attenuation image acquisition unit is configured to acquire a radiograph image of a patient. The radiograph X-ray attenuation image acquisition unit is configured to provide the radiograph image to the processing unit. The at least one sensor is configured to acquire sensor data of the patient. The at least one sensor is configured to provide the sensor data to the processing unit. The processing unit is configured to determine a magnitude and direction of movement of the patient during a time of acquisition of the radiograph image. The determination of the magnitude and direction of movement of the patient comprises utilization of the sensor data. The processing unit is configured to post-process the radiograph image comprising utilization of the determined magnitude and direction of movement of the patient during the time of acquisition of the radiograph image.

According to an example, the at least one sensor comprises one or more of: a 3D camera; a camera; a pair of stereo cameras; one or more ultrasound sensors; one or more radar sensors; one or more accelerometers attached to the patient.

According to an example, the sensor data comprises sensor data of the patient during the time of acquisition of the radiograph image of the patient.

According to an example, the sensor data of the patient comprises a plurality of sensor data acquired at different time points. The processing unit is configured to determine the magnitude and direction of movement of the patient during the time of acquisition of the radiograph image. This determination of the magnitude and direction of movement of the patient during the time of acquisition of the radiograph image can comprises interpolation and/or extrapolation of the plurality of sensor data.

In an example, the sensor data of the patient comprises a plurality of sensor data acquired at different time points. The processing unit is configured to determine the magnitude and direction of movement of the patient during the time of acquisition of the radiograph image. This determination of the magnitude and direction of movement of the patient during the time of acquisition of the radiograph image can comprises an averaging of the plurality of sensor data.

According to an example, post-processing of the radiograph image comprises a processing of the radiograph image by an image processing algorithm implemented by the processing unit. The processing unit is configured to modify at least one parameter of the image processing algorithm on the basis of the determined magnitude and direction of movement of the patient during the time of acquisition of the radiograph image.

According to an example, post-processing of the radiograph image comprises a processing of the radiograph image by an image processing algorithm implemented by the processing unit. The processing unit is configured to select at least one parameter of the image processing algorithm on the basis of the determined magnitude and direction of movement of the patient during the time of acquisition of the radiograph image.

According to an example, the image processing algorithm comprises a noise reduction algorithm, an image sharpening algorithm, a contrast enhancement algorithm, or a de-blurring algorithm.

According to an example, the at least one parameter comprises a local strength and direction of at least one kernel of the image processing algorithm.

According to an example, the processing unit is configured to estimate 2D optical flow or 3D optical flow in the image plane to determine the magnitude and direction of movement of the patient during the time of acquisition of the radiograph image.

According to an example, post-processing of the radiograph image comprises a determination of a quantitative length value through the patient.

According to an example, post-processing of the radiograph image comprises a determination of a quantitative volume value of the patient.

According to an example, the system is configured to output information that the patient has moved during the time of acquisition of the radiograph image.

According to an example, the system is configured to output the information that the patient has moved during the time of acquisition of the radiograph image when the determined magnitude of movement exceeds a threshold value.

From the above description, it will be appreciated that an apparatus with a processing unit can operate in an offline or quasi offline mode or indeed real time mode, to receive the above described radiograph image and the sensor data and determine a magnitude and direction of movement of the patient during a time of acquisition of the radiograph image and post-process the radiograph image using the determined magnitude and direction of movement of the patient during the time of acquisition of the radiograph image.

Thus, an example of an apparatus for post-processing a radiograph X-ray attenuation image comprises a processing unit 40. The processing unit is configured to receive a radiograph image of a patient acquired by a radiograph X-ray attenuation image acquisition unit 20. The processing unit is configured to receive sensor data of the patient acquired by at least one sensor 30. The processing unit is configured to determine a magnitude and direction of movement of the patient during a time of acquisition of the radiograph image. The determination of the magnitude and direction of movement of the patient comprises utilization of the sensor data. The processing unit is configured to post-process the radiograph image comprising utilization of the determined magnitude and direction of movement of the patient during the time of acquisition of the radiograph image.

In an example, the at least one sensor comprises one or more of: a 3D camera; a camera; a pair of stereo cameras; one or more ultrasound sensors; one or more radar sensors; one or more accelerometers attached to the patient.

In an example, the sensor data comprises sensor data of the patient during the time of acquisition of the radiograph image of the patient.

In an example, the sensor data of the patient comprises a plurality of sensor data acquired at different time points. The processing unit is configured to determine the magnitude and direction of movement of the patient during the time of acquisition of the radiograph image. This determination of the magnitude and direction of movement of the patient during the time of acquisition of the radiograph image can comprises interpolation and/or extrapolation of the plurality of sensor data.

In an example, the sensor data of the patient comprises a plurality of sensor data acquired at different time points. The processing unit is configured to determine the magnitude and direction of movement of the patient during the time of acquisition of the radiograph image. This determination of the magnitude and direction of movement of the patient during the time of acquisition of the radiograph image can comprises an averaging of the plurality of sensor data.

In an example, post-processing of the radiograph image comprises a processing of the radiograph image by an image processing algorithm implemented by the processing unit. The processing unit is configured to modify at least one parameter of the image processing algorithm on the basis of the determined magnitude and direction of movement of the patient during the time of acquisition of the radiograph image.

In an example, post-processing of the radiograph image comprises a processing of the radiograph image by an image processing algorithm implemented by the processing unit. The processing unit is configured to select at least one parameter of the image processing algorithm on the basis of the determined magnitude and direction of movement of the patient during the time of acquisition of the radiograph image.

In an example, the image processing algorithm comprises a noise reduction algorithm, an image sharpening algorithm, a contrast enhancement algorithm, or a de-blurring algorithm.

In example, the at least one parameter comprises a local strength and direction of at least one kernel of the image processing algorithm.

In an example, the processing unit is configured to estimate 2D optical flow or 3D optical flow in the image plane to determine the magnitude and direction of movement of the patient during the time of acquisition of the radiograph image.

In an example, post-processing of the radiograph image comprises a determination of a quantitative length value through the patient.

In an example, post-processing of the radiograph image comprises a determination of a quantitative volume value of the patient.

In an example, the system is configured to output information that the patient has moved during the time of acquisition of the radiograph image.

In an example, the system is configured to output the information that the patient has moved during the time of acquisition of the radiograph image when the determined magnitude of movement exceeds a threshold value.

Fig. 2 shows an example of an X-ray imaging method 100 in its basic steps. The method 100 comprises:
in an acquiring step 110, also referred to as step a), acquiring a radiograph image of a patient with an radiograph X-ray attenuation image acquisition unit;
in a providing step 120, also referred to as step b), providing the radiograph image to a processing unit;
in an acquiring step 130, also referred to as step c), acquiring sensor data of the patient with at least one sensor;
in a providing step 140, also referred to as step d), providing the sensor data to the processing unit;
in a determining step 150, also referred to as step e), determining by the processing unit a magnitude and direction of movement of the patient during a time of acquisition of the radiograph image, the determining comprising utilizing the sensor data; and
in a post-processing step 160, also referred to as step f), post-processing by the processing unit the radiograph image comprising utilizing the determined magnitude and direction of movement of the patient during the time of acquisition of the radiograph image.

Certain steps can occur at different times, for example step c) can occur before, at the same time as, during, and/or after step a).

Also, sensor data can refer to a single sensor data acquisition or a series of sensor data acquisitions, that can be at different times.

Sensor data can also referred to sensor data acquired by different sensors contemporaneously, thereby providing for sensor data redundancy and enabling sensor data from different sensors to complement one another and provide for more accurate quantitative information.

In an example, the at least one sensor comprises one or more of: a 3D camera; a camera; a pair of stereo cameras; one or more ultrasound sensors; one or more radar sensors; one or more accelerometers attached to the patient.

In an example, the sensor data comprises sensor data of the patient during the time of acquisition of the radiograph image of the patient.

In an example, the sensor data of the patient comprises a plurality of sensor data acquired at different time points. The method can then comprise determining by the processing unit the magnitude and direction of movement of the patient during the time of acquisition of the radiograph image comprising interpolation and/or extrapolation of the plurality of sensor data.

In an example, step f) comprises processing of the radiograph image by an image processing algorithm implemented by the processing unit, and modifying by the processing unit at least one parameter of the image processing algorithm on the basis of the determined magnitude and direction of movement of the patient during the time of acquisition of the radiograph image.

In an example, step f) comprises processing of the radiograph image by an image processing algorithm implemented by the processing unit, and selecting by the processing unit at least one parameter of the image processing algorithm on the basis of the determined magnitude and direction of movement of the patient during the time of acquisition of the radiograph image.

In an example, the image processing algorithm comprises a noise reduction algorithm, an image sharpening algorithm, a contrast enhancement algorithm, or a de-blurring algorithm. The image processing algorithm can utilise one or more of these algorithms.

In an example, the at least one parameter comprises a local strength and direction of at least one kernel of the image processing algorithm.

In an example, step e) comprises estimating by the processing unit 2D optical flow or 3D optical flow in the image plane to determine the magnitude and direction of movement of the patient during the time of acquisition of the radiograph image.

In an example, step f) comprises determining a quantitative length value through the patient.

In an example, step f) comprises determining a quantitative volume value of the patient.

In an example, the method comprises outputting information that the patient has moved during the time of acquisition of the radiograph image.

In an example, the method comprises outputting the information that the patient has moved during the time of acquisition of the radiograph image when the determined magnitude of movement exceeds a threshold value.

From the above description, it would be appreciated that such a method can operate in an offline, quasi offline or indeed real time mode, without requiring to be intrinsically linked with acquisition of the radiograph or acquisition of the sensor data as such. In other words, such a method comprises a number of, but not all, the method steps described above and as shown in Fig. 2.

Thus, an example of a method of post-processing a radiograph X-ray attenuation image comprises:
in a providing step 120, also referred to as step b), providing a radiograph image of a patient to a processing unit, wherein the radiograph image of the patient was acquired by a radiograph X-ray attenuation image acquisition unit;
in a providing step 140, also referred to as step d), providing sensor data of the patient to the processing unit, wherein the sensor data was acquired by at least one sensor;
in a determining step 150, also referred to as step e), determining by the processing unit a magnitude and direction of movement of the patient during a time of acquisition of the radiograph image, the determining comprising utilizing the sensor data; and
in a post-processing step 160, also referred to as step f), post-processing by the processing unit the radiograph image comprising utilizing the determined magnitude and direction of movement of the patient during the time of acquisition of the radiograph image.

Sensor data can refer to a single sensor data acquisition or a series of sensor data acquisitions, that can be at different times.

Sensor data can also referred to sensor data acquired by different sensors contemporaneously, thereby providing for sensor data redundancy and enabling sensor data from different sensors to complement one another and provide for more accurate quantitative information.

In an example, the at least one sensor comprises one or more of: a 3D camera; a camera; a pair of stereo cameras; one or more ultrasound sensors; one or more radar sensors; one or more accelerometers attached to the patient.

In an example, the sensor data comprises sensor data of the patient during the time of acquisition of the radiograph image of the patient.

In an example, the sensor data of the patient comprises a plurality of sensor data acquired at different time points. The method can then comprise determining by the processing unit the magnitude and direction of movement of the patient during the time of acquisition of the radiograph image comprising interpolation and/or extrapolation of the plurality of sensor data.

In an example, step f) comprises processing of the radiograph image by an image processing algorithm implemented by the processing unit, and modifying by the processing unit at least one parameter of the image processing algorithm on the basis of the determined magnitude and direction of movement of the patient during the time of acquisition of the radiograph image.

In an example, step f) comprises processing of the radiograph image by an image processing algorithm implemented by the processing unit, and selecting by the processing unit at least one parameter of the image processing algorithm on the basis of the determined magnitude and direction of movement of the patient during the time of acquisition of the radiograph image.

In an example, the image processing algorithm comprises a noise reduction algorithm, an image sharpening algorithm, a contrast enhancement algorithm, or a de-blurring algorithm. The image processing algorithm can utilise one or more of these algorithms.

In an example, the at least one parameter comprises a local strength and direction of at least one kernel of the image processing algorithm.

In an example, step e) comprises estimating by the processing unit 2D optical flow or 3D optical flow in the image plane to determine the magnitude and direction of movement of the patient during the time of acquisition of the radiograph image.

In an example, step f) comprises determining a quantitative length value through the patient.

In an example, step f) comprises determining a quantitative volume value of the patient.

In an example, the method comprises outputting information that the patient has moved during the time of acquisition of the radiograph image.

In an example, the method comprises outputting the information that the patient has moved during the time of acquisition of the radiograph image when the determined magnitude of movement exceeds a threshold value.

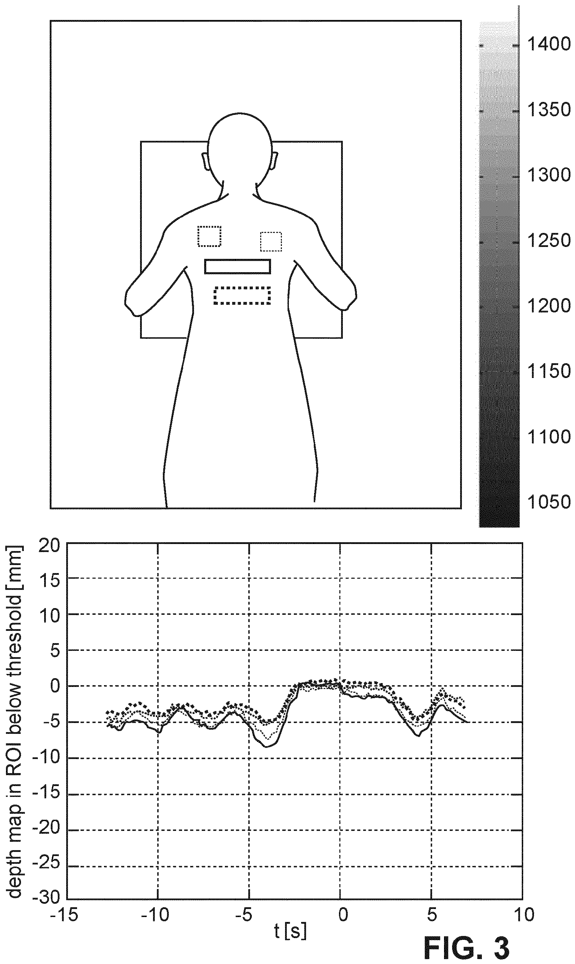

The X-ray imaging system and apparatus and X-ray imaging methods are now described with respect to specific embodiments, where reference is made to Fig. 3.

In recent times, cameras have been included in medical imaging systems to monitor the patient and automate the workflow. For example, a 3D camera is utilized for automatic adjustment of the collimation window. However, the inventors realised that for a radiography system, data from the 3D camera, or indeed from other sensors, could be utilised to determine the direction and magnitude of the patient during the acquisition window, or acquisition time, of the radiography system. This motion of the patient during the acquisition time causes blurring of the resultant radiogram or radiography image, which up until now has been analysed itself in order to determine a blurring kernel of an image processing algorithm, but now the motion information determined from the actual sensed to motion of the patient can be utilised to determine the required blurring kernel.

In an exemplar radiography system, the camera is mounted in the tube head, i.e. directed at the patient from a perspective similar to the x-ray tube. The camera could be a RGB camera, a 3D camera (e.g. structured light or ToF) or any other range sensor that is able to assess local changes in the patient position, e.g. distributed ultra sound sensors. Radar sensors or even accelerometers attached to the patient). This sensor captures information about the patient and the image acquisition procedure that is not available in the radiographic image. In particular, the camera can capture the amount and direction of patient motion during the acquisition. This local motion information, as captured by a camera (or other sensor), is then used to guide the selection of sharpening/de-blurring operators in an image post-processing step.

Temporal information from the camera is used to quantify the amount of local motion in the scene at the time of the x-ray image acquisition. An estimation of the 2D optical flow in the image plane (or the 3D optical flow) is then used to select the local strength and direction of de-blurring kernels in the image post-processing step. Moreover, the amount of detected motion can be delivered to the radiologist, and if necessary this can be provided only when it has exceeded a threshold. The threshold can be user defined or defined at a level where motion induced would be expected to begin to compromise the image integrity.

If a 3D camera is used, the z-component of the local 3D patient motion can be estimated from a sequence of depth images. An example of detected breathing motion and breath-hold at t=0 (image acquisition time) is shown in Fig. 3. In Fig. 3 a schematic representation of an image shows regions of interests, where motion information is being determined from the 3D camera data. A plot then shows the average depth values of these ROIs as a function of time. The average depth value inside the ROIs represent the local motion of the patient's back due to breathing. The example shows different amplitudes of the respiratory motion for the upper and lower back region. It is important to note that this example only shows the (integrated) z-component of the 3D motion vectors. However, this information enables de-blurring correction to be applied, with different de-blurring kernel strengths, at these ROIs dependent upon the magnitude of the motion during the acquisition. The local motion within the image plane (x/y component) can also be assessed from an RGB-camera stream.

Simulated radiographies, with and without motion of the patient, can be used to learn the appearance of motion blur in the radiography using a convolutional neural network.

The present system and method described here therefore enables transformation of radiographs acquired when the patient was in motion towards that of radiographs acquired without patient motion. This can be done in real-time or quasi real-time during acquisition of the radiograph or carried out for example later by processing the previously acquired radiograph and sensor data to determine the magnitude and direction of movement and post-process the radiograph accordingly.

In another exemplary embodiment, a computer program or computer program element is provided that is characterized by being configured to execute the method steps of the method according to one of the preceding embodiments, on an appropriate system.

The computer program element might therefore be stored on a computer unit, which might also be part of an embodiment. This computing unit may be configured to perform or induce performing of the steps of the method described above. Moreover, it may be configured to operate the components of the above described apparatus and/or system. The computing unit can be configured to operate automatically and/or to execute the orders of a user. A computer program may be loaded into a working memory of a data processor. The data processor may thus be equipped to carry out the method according to one of the preceding embodiments.

This exemplary embodiment of the invention covers both, a computer program that right from the beginning uses the invention and computer program that by means of an update turns an existing program into a program that uses invention.

Further on, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An X-ray imaging system (10), comprising:
- a radiograph X-ray attenuation image acquisition unit (20);
- at least one sensor (30); and
- a processing unit (40);
wherein, the radiograph X-ray attenuation image acquisition unit is configured to acquire a radiograph image of a patient, wherein the radiograph image consists of only a single x-ray projection image;
wherein, the radiograph X-ray attenuation image acquisition unit is configured to provide the radiograph image to the processing unit;
wherein, the at least one sensor is configured to acquire sensor data of the patient;
wherein, the at least one sensor is configured to provide the sensor data to the processing unit;
wherein, the processing unit is configured to determine a magnitude and direction of movement of the patient during a time of acquisition of the radiograph image, the determination comprising utilization of the sensor data; and
wherein, the processing unit is configured to post-process the radiograph image comprising utilization of the determined magnitude and direction of movement of the patient during the time of acquisition of the radiograph image.

2. System according to claim 1, wherein the at least one sensor comprises one or more of: a 3D camera; a camera; a pair of stereo cameras; one or more ultrasound sensors; one or more radar sensors; one or more accelerometers attached to the patient.

3. System according to any of claims 1-2, wherein, the sensor data comprises sensor data of the patient during the time of acquisition of the radiograph image of the patient.

4. System according to any of claims 1-3, wherein the sensor data of the patient comprises a plurality of sensor data acquired at different time points, and wherein the processing unit is configured to determine the magnitude and direction of movement of the patient during the time of acquisition of the radiograph image comprising interpolation and/or extrapolation of the plurality of sensor data.

5. System according to any of claims 1-4, wherein post-processing of the radiograph image comprises a processing of the radiograph image by an image processing algorithm implemented by the processing unit, and wherein the processing unit is configured to modify at least one parameter of the image processing algorithm on the basis of the determined magnitude and direction of movement of the patient during the time of acquisition of the radiograph image.

6. System according to any of claims 1-5, wherein post-processing of the radiograph image comprises a processing of the radiograph image by an image processing algorithm implemented by the processing unit, and wherein the processing unit is configured to select at least one parameter of the image processing algorithm on the basis of the determined magnitude and direction of movement of the patient during the time of acquisition of the radiograph image.

7. System according to any of claims 5-6, wherein the image processing algorithm comprises a noise reduction algorithm, an image sharpening algorithm, a contrast enhancement algorithm, or a de-blurring algorithm.

8. System according to any of claims 5-7, wherein the at least one parameter comprises a local strength and direction of at least one kernel of the image processing algorithm.

9. System according to any of claims 1-8, wherein the processing unit is configured to estimate 2D optical flow or 3D optical flow in the image plane to determine the magnitude and direction of movement of the patient during the time of acquisition of the radiograph image.

10. System according to any of claims 1-9, wherein post-processing of the radiograph image comprises a determination of a quantitative length value through the patient; and optionally or wherein post-processing of the radiograph image comprises a determination of a quantitative volume value of the patient.

11. System according to any of claims 1-10, wherein the system is configured to output information that the patient has moved during the time of acquisition of the radiograph image; and optionally wherein the system is configured to output the information that the patient has moved during the time of acquisition of the radiograph image when the determined magnitude of movement exceeds a threshold value.

12. An apparatus for post-processing a radiograph X-ray attenuation image, comprising:
- a processing unit (40);
wherein the processing unit is configured to receive a radiograph image of a patient, wherein the radiograph image consists of only a single x-ray projection image, acquired by a radiograph X-ray attenuation image acquisition unit (20);
wherein, the processing unit is configured to receive sensor data of the patient acquired by at least one sensor (30);
wherein, the processing unit is configured to determine a magnitude and direction of movement of the patient during a time of acquisition of the radiograph image, wherein the determination of the magnitude and direction of movement of the patient comprises utilization of the sensor data; and
wherein the processing unit is configured to post-process the radiograph image comprising utilization of the determined magnitude and direction of movement of the patient during the time of acquisition of the radiograph image.

13. An X-ray imaging method (100), comprising:
a) acquiring (110) a radiograph image of a patient, wherein the radiograph image consists of only a single x-ray projection image, with an radiograph X-ray attenuation image acquisition unit;
b) providing (120) the radiograph image to a processing unit;
c) acquiring (130) sensor data of the patient with at least one sensor;
d) providing (140) the sensor data to the processing unit;
e) determining (150) by the processing unit a magnitude and direction of movement of the patient during a time of acquisition of the radiograph image, the determining comprising utilizing the sensor data; and
f) post-processing (160) by the processing unit the radiograph image comprising utilizing the determined magnitude and direction of movement of the patient during the time of acquisition of the radiograph image.

14. A method of post-processing a radiograph X-ray attenuation image, comprising:
b) providing (120) a radiograph image of a patient, wherein the radiograph consists of only a single x-ray projection image, to a processing unit, wherein the radiograph image of the patient was acquired by a radiograph X-ray attenuation image acquisition unit;
d) providing (140) sensor data of the patient to the processing unit, wherein the sensor data was acquired by at least one sensor;
e) determining (150) by the processing unit a magnitude and direction of movement of the patient during a time of acquisition of the radiograph image, the determining comprising utilizing the sensor data; and
f) post-processing (160) by the processing unit the radiograph image comprising utilizing the determined magnitude and direction of movement of the patient during the time of acquisition of the radiograph image.

15. A computer program element for controlling a system according to one of claims 1 to 11, which when executed by a processor is configured to carry out the method of claim 13, or a computer program element for controlling an apparatus according to claim 12, which when executed by a processor is configured to carry out the method of claim 14.

## Patentansprüche

1. Röntgenbildgebungssystem (10), umfassend:
- eine Röntgenstrahlabschwächung-Bildaufnahmeeinheit (20) ;
- mindestens einen Sensor (30); und
- eine Verarbeitungseinheit (40);
wobei die Röntgenstrahlabschwächung-Bildaufnahmeeinheit konfiguriert ist, um ein Röntgenbild eines Patienten aufzunehmen, wobei das Röntgenbild aus nur einem einzigen Röntgenprojektionsbild besteht;
wobei die Röntgenstrahlabschwächung-Bildaufnahmeeinheit konfiguriert ist, um das Röntgenbild an die Verarbeitungseinheit bereitzustellen;
wobei der mindestens eine Sensor konfiguriert ist, Sensordaten des Patienten aufzunehmen;
wobei der mindestens eine Sensor konfiguriert ist, um die Sensordaten an die Verarbeitungseinheit bereitzustellen;
wobei die Verarbeitungseinheit konfiguriert ist, um eine Größe und Richtung einer Bewegung des Patienten während einer Aufnahmezeit des Röntgenbilds zu bestimmen, die Bestimmung umfassend eine Verwendung der Sensordaten; und
wobei die Verarbeitungseinheit konfiguriert ist, um das Röntgenbild nachzubearbeiten, umfassend eine Verwendung der bestimmten Größe und Richtung einer Bewegung des Patienten während der Aufnahmezeit des Röntgenbilds.

2. System nach Anspruch 1, wobei der mindestens eine Sensor eines oder mehrere von Folgenden umfasst:
eine 3D-Kamera; eine Kamera; ein Paar Stereokameras; einen oder mehrere Ultraschallsensoren; einen oder mehrere Radarsensoren; einen oder mehrere Beschleunigungsmesser, die an dem Patienten angebracht sind.

3. System nach einem der Ansprüche 1-2, wobei die Sensordaten Sensordaten des Patienten während der Aufnahmezeit des Röntgenbilds des Patienten umfassen.

4. System nach einem der Ansprüche 1-3, wobei die Sensordaten des Patienten eine Vielzahl von Sensordaten umfassen, die zu unterschiedlichen Zeitpunkten aufgenommen werden, und wobei die Verarbeitungseinheit konfiguriert ist, um die Größe und Richtung einer Bewegung des Patienten während der Aufnahmezeit des Röntgenbilds durch Interpolation und/oder Extrapolation der Vielzahl von Sensordaten zu bestimmen.

5. System nach einem der Ansprüche 1-4, wobei eine Nachbearbeitung des Röntgenbilds ein Verarbeiten des Röntgenbilds durch einen Bildverarbeitungsalgorithmus umfasst, der von der Verarbeitungseinheit implementiert wird, und wobei die Verarbeitungseinheit konfiguriert ist, um mindestens einen Parameter des Bildverarbeitungsalgorithmus basierend auf der bestimmten Größe und Richtung einer Bewegung des Patienten während der Aufnahmezeit des Röntgenbilds zu modifizieren.

6. System nach einem der Ansprüche 1-5, wobei eine Nachbearbeitung des Röntgenbilds ein Verarbeiten des Röntgenbilds durch einen Bildverarbeitungsalgorithmus umfasst, der von der Verarbeitungseinheit implementiert wird, und wobei die Verarbeitungseinheit konfiguriert ist, um mindestens einen Parameter des Bildverarbeitungsalgorithmus basierend auf der bestimmten Größe und Richtung einer Bewegung des Patienten während der Aufnahmezeit des Röntgenbilds auszuwählen.

7. System nach einem der Ansprüche 5-6, wobei der Bildverarbeitungsalgorithmus einen Rauschunterdrückungsalgorithmus, einen Bildschärfungsalgorithmus, einen Kontrastverbesserungsalgorithmus oder einen Entschärfungsalgorithmus umfasst.

8. System nach einem der Ansprüche 5-7, wobei der mindestens eine Parameter eine lokale Stärke und Richtung von mindestens einem Kernel des Bildverarbeitungsalgorithmus umfasst.

9. System nach einem der Ansprüche 1-8, wobei die Verarbeitungseinheit konfiguriert ist, um den optischen 2D-Fluss oder den optischen 3D-Fluss in der Bildebene zu schätzen, um die Größe und Richtung einer Bewegung des Patienten während der Aufnahmezeit des Röntgenbilds zu bestimmen.

10. System nach einem der Ansprüche 1-9, wobei ein Nachbearbeiten des Röntgenbilds eine Bestimmung eines quantitativen Längenwerts durch den Patienten umfasst; und optional oder wobei ein Nachbearbeiten des Röntgenbildes eine Bestimmung eines quantitativen Volumenwerts des Patienten umfasst.

11. System nach einem der Ansprüche 1-10, wobei das System konfiguriert ist, um Informationen darüber auszugeben, dass sich der Patient während der Aufnahmezeit des Röntgenbilds bewegt hat; und wobei das System optional konfiguriert ist, um die Informationen darüber auszugeben, dass sich der Patient während der Aufnahmezeit des Röntgenbilds bewegt hat, wenn die bestimmte Größe einer Bewegung einen Schwellenwert überschreitet.

12. Vorrichtung zum Nachbearbeiten eines Röntgenstrahlabschwächung-Bilds, umfassend:
- eine Verarbeitungseinheit (40);
wobei die Verarbeitungseinheit konfiguriert ist, um ein Röntgenbild eines Patienten zu empfangen, wobei das Röntgenbild aus nur einem einzigen Röntgenprojektionsbild besteht, das von einer Röntgenstrahlabschwächung-Bildaufnahmeeinheit (20) aufgenommen wird;
wobei die Verarbeitungseinheit konfiguriert ist, um Sensordaten des Patienten zu empfangen, die von mindestens einem Sensor (30) aufgenommen werden;
wobei die Verarbeitungseinheit konfiguriert ist, um eine Größe und Richtung einer Bewegung des Patienten während einer Aufnahmezeit des Röntgenbilds zu bestimmen, wobei die Bestimmung der Größe und Richtung einer Bewegung des Patienten eine Verwendung der Sensordaten umfasst; und
wobei die Verarbeitungseinheit konfiguriert ist, um das Röntgenbild nachzubearbeiten, umfassend eine Verwendung der bestimmten Größe und Richtung einer Bewegung des Patienten während der Aufnahmezeit des Röntgenbilds.

13. Röntgenbildgebungsverfahren (100), umfassend:
a) Aufnehmen (110) eines Röntgenbilds eines Patienten, wobei das Röntgenbild aus nur einem einzigen Röntgenprojektionsbild besteht, mit einer Röntgenstrahlabschwächung-Bildaufnahmeeinheit;
b) Bereitstellen (120) des Röntgenbilds an eine Verarbeitungseinheit;
c) Aufnehmen (130) von Sensordaten des Patienten mit mindestens einem Sensor;
d) Bereitstellen (140) der Sensordaten an die Verarbeitungseinheit;
e) Bestimmen (150), durch die Verarbeitungseinheit, einer Größe und Richtung einer Bewegung des Patienten während der Aufnahmezeit des Röntgenbilds, das Bestimmen umfassend ein Verwenden der Sensordaten; und
f) Nachbearbeiten (160), durch die Verarbeitungseinheit, des Röntgenbilds, wobei die bestimmte Größe und Richtung einer Bewegung des Patienten während der Aufnahmezeit des Röntgenbilds verwendet wird.

14. Verfahren zum Nachbearbeiten eines Röntgenstrahlabschwächung-Bilds, umfassend:
b) Bereitstellen (120) eines Röntgenbilds eines Patienten, wobei das Röntgenbild aus nur einem einzigen Röntgenprojektionsbild besteht, an eine Verarbeitungseinheit, wobei das Röntgenbild des Patienten von einer Röntgenstrahlabschwächung-Bildaufnahmeeinheit aufgenommen wird;
d) Bereitstellen (140) von Sensordaten des Patienten an die Verarbeitungseinheit, wobei die Sensordaten von mindestens einem Sensor aufgenommen werden;
e) Bestimmen (150), durch die Verarbeitungseinheit, einer Größe und Richtung einer Bewegung des Patienten während der Aufnahmezeit des Röntgenbilds, das Bestimmen umfassend ein Verwenden der Sensordaten; und
f) Nachbearbeiten (160), durch die Verarbeitungseinheit, des Röntgenbilds, wobei die bestimmte Größe und Richtung einer Bewegung des Patienten während der Aufnahmezeit des Röntgenbilds verwendet wird.

15. Computerprogrammelement zum Steuern eines Systems nach einem der Ansprüche 1 bis 11, das, wenn es von einem Prozessor ausgeführt wird, konfiguriert ist, um das Verfahren nach Anspruch 13 auszuführen, oder ein Computerprogrammelement zum Steuern einer Vorrichtung nach Anspruch 12, das, wenn es von einem Prozessor ausgeführt wird, konfiguriert ist, um das Verfahren nach Anspruch 14 auszuführen.

## Revendications

1. Système d'imagerie à rayons X (10), comprenant:
- une unité d'acquisition d'image d'atténuation de rayons X de radiographie (20);
- au moins un capteur (30); et
- une unité de traitement (40); dans lequel l'unité d'acquisition d'image d'atténuation de rayons X de radiographie est configurée pour acquérir une image de radiographie d'un patient, dans lequel l'image de radiographie se compose uniquement d'une seule image de projection de rayons X;
dans lequel l'unité d'acquisition d'image d'atténuation de rayons X de radiographie est configurée pour fournir l'image de radiographie à l'unité de traitement;
dans lequel l'au moins un capteur est configuré pour acquérir des données de capteur du patient; dans lequel l'au moins un capteur est configuré pour fournir les données de capteur à l'unité de traitement;
dans lequel l'unité de traitement est configurée pour déterminer une amplitude et une direction de mouvement du patient pendant un temps d'acquisition de l'image de radiographie, la détermination comprenant l'utilisation des données de capteur; et
dans lequel l'unité de traitement est configurée pour post-traiter l'image de radiographie comprenant l'utilisation de l'amplitude et de la direction de mouvement du patient déterminées pendant le temps d'acquisition de l'image de radiographie.

2. Système selon la revendication 1, dans lequel l'au moins un capteur comprend un ou plusieurs parmi:
une caméra 3D; une caméra; une paire de caméras stéréo; un ou plusieurs capteurs à ultrasons; un ou plusieurs capteurs radar; un ou plusieurs accéléromètres attachés au patient.

3. Système selon l'une quelconque des revendications 1-2, dans lequel les données de capteur comprennent des données de capteur du patient pendant le temps d'acquisition de l'image de radiographie du patient.

4. Système selon l'une quelconque des revendications 1-3, dans lequel les données de capteur du patient comprennent une pluralité de données de capteur acquises à différents instants, et dans lequel l'unité de traitement est configurée pour déterminer l'amplitude et la direction de mouvement du patient pendant le temps d'acquisition de l'image de radiographie comprenant une interpolation et/ou une extrapolation de la pluralité de données de capteur.

5. Système selon l'une quelconque des revendications 1-4, dans lequel le post-traitement de l'image de radiographie comprend un traitement de l'image de radiographie par un algorithme de traitement d'image mis en œuvre par l'unité de traitement, et dans lequel l'unité de traitement est configurée pour modifier au moins un paramètre de l'algorithme de traitement d'image sur la base de l'amplitude et de la direction de mouvement du patient déterminées pendant le temps d'acquisition de l'image de radiographie.

6. Système selon l'une quelconque des revendications 1-5, dans lequel le post-traitement de l'image de radiographie comprend un traitement de l'image de radiographie par un algorithme de traitement d'image mis en œuvre par l'unité de traitement, et dans lequel l'unité de traitement est configurée pour sélectionner au moins un paramètre de l'algorithme de traitement d'image sur la base de l'amplitude et de la direction de mouvement du patient déterminées pendant le temps d'acquisition de l'image de radiographie.

7. Système selon l'une quelconque des revendications 5-6, dans lequel l'algorithme de traitement d'image comprend un algorithme de réduction de bruit, un algorithme d'accentuation d'image, un algorithme d'amélioration de contraste ou un algorithme de suppression du flou.

8. Système selon l'une quelconque des revendications 5-7, dans lequel l'au moins un paramètre comprend une force et une direction locales d'au moins un noyau de l'algorithme de traitement d'image.

9. Système selon l'une quelconque des revendications 1-8, dans lequel l'unité de traitement est configurée pour estimer le flux optique 2D ou le flux optique 3D dans le plan image pour déterminer l'amplitude et la direction de mouvement du patient pendant le temps d'acquisition de l'image de radiographie.

10. Système selon l'une quelconque des revendications 1-9, dans lequel le post-traitement de l'image de radiographie comprend une détermination d'une valeur de longueur quantitative à travers le patient; et facultativement ou dans lequel le post-traitement de l'image de radiographie comprend une détermination d'une valeur de volume quantitative du patient.

11. Système selon l'une quelconque des revendications 1-10, dans lequel le système est configuré pour émettre des informations indiquant que le patient a bougé pendant le temps d'acquisition de l'image de radiographie; et facultativement dans lequel le système est configuré pour émettre les informations selon lesquelles le patient a bougé pendant le temps d'acquisition de l'image de radiographie lorsque l'amplitude de mouvement déterminée dépasse une valeur seuil.

12. Appareil pour le post-traitement d'une image d'atténuation de rayons X de radiographie, comprenant:
- une unité de traitement (40);
dans lequel l'unité de traitement est configurée pour recevoir une image de radiographie d'un patient, dans lequel l'image de radiographie se compose uniquement d'une seule image de projection de rayons X, acquise par une unité d'acquisition d'image d'atténuation de rayons X de radiographie (20);
dans lequel l'unité de traitement est configurée pour recevoir des données de capteur du patient acquises par au moins un capteur (30);
dans lequel, l'unité de traitement est configurée pour déterminer une amplitude et une direction de mouvement du patient pendant un temps d'acquisition de l'image de radiographie, dans lequel la détermination de l'amplitude et de la direction de mouvement du patient comprend l'utilisation des données de capteur; et
dans lequel l'unité de traitement est configurée pour post-traiter l'image de radiographie comprenant l'utilisation de l'amplitude et de la direction de mouvement du patient déterminées pendant le temps d'acquisition de l'image de radiographie.

13. Procédé d'imagerie par rayons X (100), consistant à:
a) acquérir (110) une image de radiographie d'un patient, dans lequel l'image de radiographie se compose uniquement d'une seule image de projection de rayons X, avec une unité d'acquisition d'image d'atténuation de rayons X de radiographie;
b) fournir (120) l'image de radiographie à une unité de traitement;
c) acquérir (130) des données de capteur du patient avec au moins un capteur;
d) fournir (140) les données de capteur à l'unité de traitement;
e) déterminer (150) par l'unité de traitement une amplitude et une direction de mouvement du patient pendant un temps d'acquisition de l'image de radiographie, la détermination comprenant l'utilisation des données de capteur; et
f) post-traiter (160) par l'unité de traitement l'image de radiographie comprenant l'utilisation de l'amplitude et de la direction de mouvement du patient déterminées pendant le temps d'acquisition de l'image de radiographie.

14. Procédé de post-traitement d'une image d'atténuation de rayons X de radiographie, consistant à:
b) fournir (120) une image de radiographie d'un patient, dans lequel la radiographie se compose uniquement d'une seule image de projection de rayons X, à une unité de traitement, dans lequel l'image de radiographie du patient a été acquise par une unité d'acquisition d'image d'atténuation de rayons X de radiographie;
d) fournir (140) des données de capteur du patient à l'unité de traitement, dans lequel les données de capteur ont été acquises par au moins un capteur;
e) déterminer (150) par l'unité de traitement une amplitude et une direction de mouvement du patient pendant un temps d'acquisition de l'image de radiographie, la détermination comprenant l'utilisation des données de capteur; et
f) post-traiter (160) par l'unité de traitement l'image de radiographie comprenant l'utilisation de l'amplitude et de la direction de mouvement du patient déterminées pendant le temps d'acquisition de l'image de radiographie.

15. Élément de programme informatique pour commander un système selon l'une quelconque des revendications 1 à 11 qui, lorsqu'il est exécuté par un processeur, est configuré pour mettre en œuvre le procédé de la revendication 13, ou élément de programme informatique pour commander un appareil selon la revendication 12 qui, lorsqu'il est exécuté par un processeur, est configuré pour mettre en œuvre le procédé de la revendication 14.
